**Europäisches Patentamt**

**European Patent Office**

**Office européen des brevets**

(19)

(11) Veröffentlichungsnummer: **0 204 100**
**B1**

(12) # EUROPÄISCHE PATENTSCHRIFT

(45) Veröffentlichungstag der Patentschrift: **04.07.90**

(51) Int. Cl.⁵: **G 01 N 3/02**

(21) Anmeldenummer: **86104637.3**

(22) Anmeldetag: **04.04.86**

(54) **Haltevorrichtung.**

(30) Priorität: **03.06.85 DE 8516163 u**

(43) Veröffentlichungstag der Anmeldung:
**10.12.86 Patentblatt 86/50**

(45) Bekanntmachung des Hinweises auf die
Patenterteilung:
**04.07.90 Patentblatt 90/27**

(84) Benannte Vertragsstaaten:
**DE FR GB NL**

(56) Entgegenhaltungen:
**DE-A-3 130 512**
**DE-B-2 812 257**
**DE-U-7 808 575**
**US-A-4 022 056**
**US-A-4 393 717**
**US-A-4 542 646**

(73) Patentinhaber: **Erweka Apparatebau GmbH**
**Ottostrasse 20-22**
**D-6056 Heusenstamm (DE)**

(72) Erfinder: **Schneider, Ortwin**
**Arheilgerstrasse 39**
**D-6108 Weiterstadt (DE)**
Erfinder: **Georgitsis, Nikolaos**
**Kolpingstrasse 34**
**D-6056 Heusenstamm (DE)**

(74) Vertreter: **Patentanwälte Kirschner & Grosse**
**Forstenrieder Allee 59**
**D-8000 München 71 (DE)**

Courier Press, Leamington Spa, England.

## Beschreibung

Die Erfindung bezieht sich auf eine Haltevorrichtung für Prüfkörper, insbesondere Tabletten und dergleichen.

Aus dem DE-GM 84 02 581 ist es bekannt, Prüfkörper in Form von Tabletten in einer Prüfeinrichtung hinsichtlich des Gewichts, der Dicke und der Bruchfestigkeit zu prüfen. Die Prüfkörper werden zu diesem Zweck mittels einer Transportvorrichtung intermittierend zu einer Waage, zu einem Dickenmeßgerät und zu einem Bruchmeßgerät transportiert. Insbesondere bei der Prüfung durch das Bruchmeßgerät kann infolge der unterschiedlichen Formen der Prüfkörper der Fall auftreten, daß sich ein Prüfkörper nicht in der gewünschten Richtung ausrichtet und damit fehlerhafte Messungen auftreten können. Dies kann insbesondere dann auftreten, wenn die Prüfkörper stäbchenförmig, länglich, oval oder herzförmig ausgebildet sind und als Haltevorrichtung lediglich eine Kante der Transportvorrichtung vorgesehen ist.

Der Erfindung liegt daher die Aufgabe zugrunde, eine Haltevorrichtung anzugeben, mittels der ein genaues Ausrichten und/oder Festhalten von Prüfkörpern mit unterschiedlichen Formen möglich ist.

Erfindungsgemäß wird die Aufgabe bei der Haltevorrichtung der eingangs genannten Art durch die in Anspruch 1 angegebenen Merkmale gelöst.

Die Haltevorrichtung gemäß der Erfindung hat den Vorteil, daß ohne große Rüstzeiten die jeweils geeigneten, an die Form des jeweiligen Prüfkörpers angepaßten Niederhalter ausgewählt werden. Die Auswahl erfolgt insbesondere selbsttätig, wobei die Haltevorrichtung durch eine Steuereinheit, die als Computer ausgebildet sein kann, in Abhängigkeit von den zu prüfenden Prüfkörpern ausgewählt wird.

Die Haltevorrichtung weist einen besonders einfachen Aufbau auf, wenn die Halterung an dem Trageteil vorzugsweise drehbar befestigt ist und die Niederhalter kreisförmig an der Halterung angeordnet sind. Es ist auch möglich, die Halterung linear auszubilden und die Niederhalter nebeneinander anzuordnen.

Eine bevorzugte Ausführungsform der Antriebsanordnung enthält einen Elektromotor, der an der Halterung angeordnet ist und der über ein Getriebe, vorzugsweise ein Kegelradgetriebe, mit dem Trageteil verbunden ist, so daß es sich gemeinsam mit der Halterung bewegt. Der Elektromotor kann jedoch auch am Trageteil befestigt sein und die Halterung antreiben.

Ein einfacher Aufbau der Absenkeinrichtung wird bei einer bevorzugten Ausführungsform der Erfindung durch einen am Trageteil angeordneten Elektromotor erreicht, der an seiner Welle mit einem Exzenter versehen ist, der bei einer Drehung des Elektromotors über ein am Niederhalter angeodnetes Rad diesen gegen die Kraft einer Rückstellfeder absenkt. Das Anheben des Niederhalters erfolgt bei einer weiteren Drehung des Exzenters mittels der Rückstellfeder, die das Rad gegen den Exzenter drückt.

Die Niederhalter weisen an ihrem unteren Ende jeweils einen für den jeweiligen Anwendungsfall geeigneten Greifer, der den Prüfkörper erfaßt und ausrichtet oder ein Kissen aus elastischem Werkstoff auf, das den Prüfkörper gegen eine Grundplatte drückt. Das Kissen wird insbesondere dann verwendet, wenn linsenförmige oder kugelförmige Prüfkörper verwendet werden.

Für die genaue Positionierung der Niederhalter in ihrer jeweiligen Arbeitsstellung ist es vorteilhaft, wenn die Positionsgeber vorgesehen sind. Auch erweist es sich als zweckmäßig, zur Arretierung der Halterung in einer Nullstellung einen Stopmagneten vorzusehen, der bei einer bevorzugten Ausfürungsform am Trageteil angeordnet ist und dessen Anker nur in der Nullstellung in eine Ausnehmung der Halterung eingreift.

Im folgenden wird ein Ausführungsbeispiel der Haltevorrichtung gemäß der Erfindung anhand von Zeichnungen näher erläutert.

Es zeigen:

Fig. 1 ein Schnittbild der Haltevorrichtung,

Fig. 2 eine Draufsicht auf in einer Halterung angeordnete Niederhalter,

Fig. 3 einen mit einem Greifer versehenen Niederhalter,

Fig. 4 einen mit einem Kissen versehenen Niederhalter.

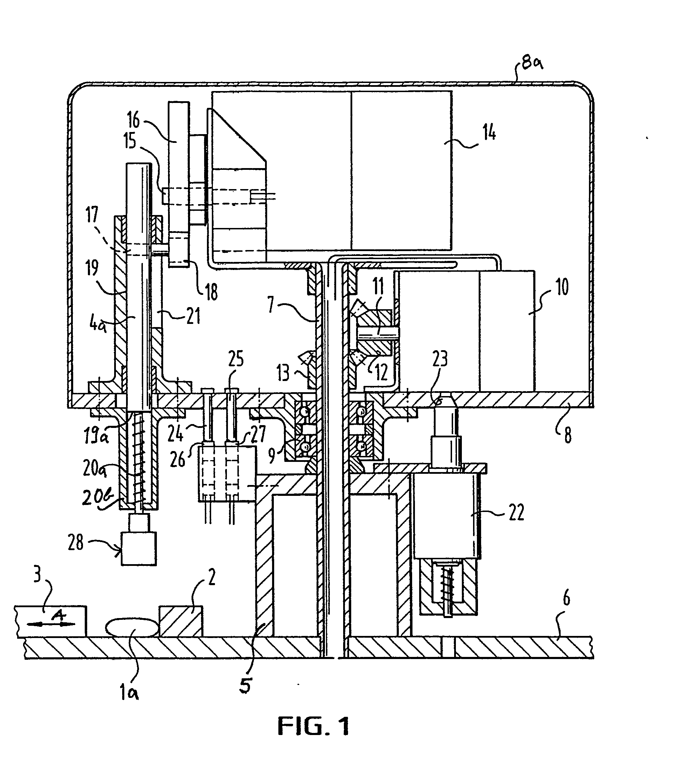

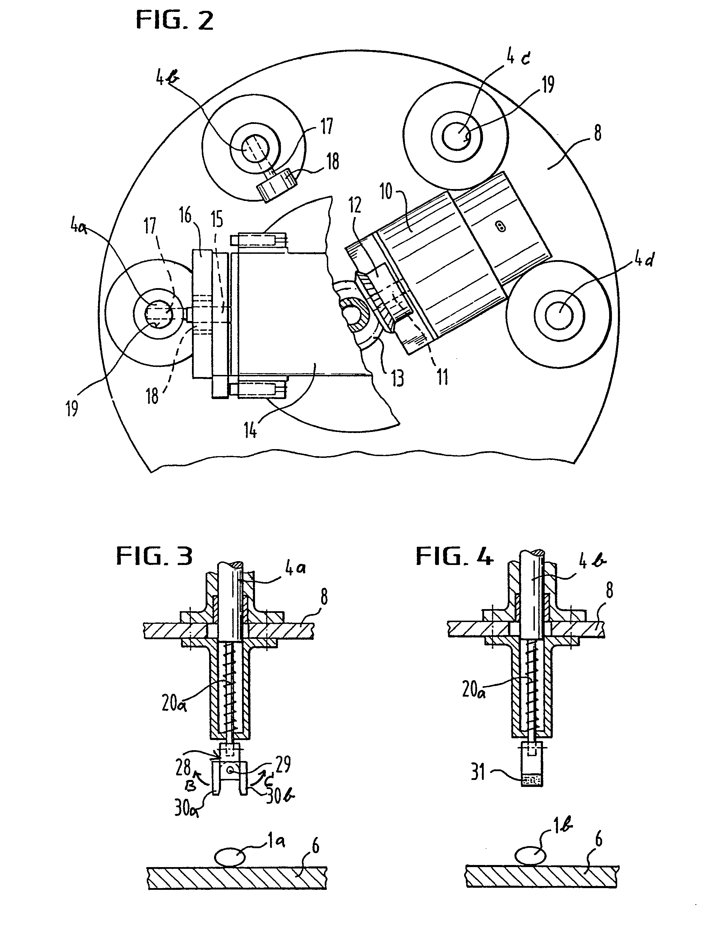

In Fig. 1 ist die Haltevorrichtung in einer Position bei einer Prüfeinrichtung gezeigt, mit der die Bruchfestigkeit von Prüfkörpern 1a gemessen wird, die als längliche Tabletten ausgebildet sind, wobei die große Achse des Prüfkörpers 1a in Fig. 1 und die kleine Achse - 180° zu der langen Achse - in Fig. 3 gezeigt ist. Die Prüfeinrichtung weist eine Grundplatte 6, ein Widerlager 2 und eine Stempel 3 auf, der in Richtung des Pfeiles A (Fig 1) mit Hilfe eines Motors (nicht gezeigt) bewegbar ist und mit einer Kraftmeßeinrichtung (nicht gezeigt) verbunden ist, die die Kraft messen kann, die von dem Stempel 3 auf den Prüfkörper 1a ausgeübt wird. Gemessen werden soll die Kraft, die notwendig ist, umden Prüfkörper 1a in Richtung der langen Achse (Fig 1) zu zerbrechen. Um diese Messung ausführen zu können, werden die Prüfkörper mit einer Transporteinrichtung, wie sie beispielsweise in dem DE-GM 84 02 581 gezeigt ist, in eine Position zwischen dem Widerlager 2 und dem Stempel 3 transportiert. Anschließend wird ein geeigneter Niederhalter 4a auf den Prüfkörper 1a abgesenkt, um diesen mit seiner langen Achse in Richtung des Pfeiles A auszurichten und ihn in dieser Position zu halten. Danach wird eine Kraft in Richtung des Pfeiles A über den Stempel 3 auf den Prüfkörper 1a ausgeübt, die ausreicht, um den Prüfkörper 1a zu zerbrechen, und diese Kraft wird unmittelbar vor dem Bruch des Prüfkörpers 1a gemessen. Die Prüfeinrichtung als solche ist die gleiche für alle verschiedenen Formen von Prüfkörpern, d.h. für scheibenförmige, linsenförmige, kugelförmige, stäbchenförmige, längliche, ovale oder herzförimge Prüfkörper. Auch verschiedene Größen dieser verschiedenen Typen von Prüfkörpern werden von ein und derselben Prüfeinrichtung verarbeitet. Es ist daher erforderlich, geeignete Niederhalter für Prüfkörper verschiedener Formen und Größen zur Verfügung zu haben, wie noch beschrieben wird.

Die Haltevorrichtung weist ein Gestell mit einem Ständer 5, der an der Grundplatte 6 befestigt ist, und eine Säule 7 auf, die an ihrem unteren Ende in der Grundplatte 6 verankert ist und von dem Ständer 5 an eine Stelle oberhalb der Grundplatte 6 abgestützt wird. Der Ständer 5 und die Säule 7 sind in bezug auf die Grundplatte 6 ortsfest angeordnet. Die Haltevorrichtung weist ferner eine bewegliche Halterung 8 auf, die die Niederhalter 4a, 4b . . . trägt. Die Halterung 8 ist durch ein Lager 9 drehbar montiert, wobei der ortsfeste Teil des Lagers an der Säule 7 und dem Ständer 5 befestigt ist, während der bewegliche Teil des Lagers 9 an der beweglichen Halterung 8 befestigt ist. Die Halterung 8 wird durch einen Elektromotor 10 gedreht, der auf der Halterung 8 befestigt ist. Die Antriebsverbindung zwischen dem Motor 10 und der ortsfesten Säule 7 wird über ein Getriebe hergestellt, das aus zwei Kegelzahnrädern 12 und 13 besteht. Daher wird durch die Drehung der Welle 11 des Elektromotors 10 die Halterung 8 zusammen mit dem Elektromotor 10 um die Säule 7 herum gedreht.

Die Niederhalter 4a, 4b . . . sind auf der Halterung 8 auf einem Kreis um die Säule 7, d.h. unter gleichem Abstand zu der Säule 7, angeordnet. Durch Drehen der Halterung 8 um die Säule 7 kann daher einer der Niederhalter in die Betriebsstellung oberhalb eines bestimmten Testkörpers in der Prüfeinrichtung gebracht werden.

Die Niederhalter 4a, 4b . . . sind stempelförmig ausgebildet und auf der Halterung 8 derart gelagert, daß sie in senkrechter Richtung hin und her bewegbar sind. Um die Niederhalter nach unten zu bewegen, ist eine Absenkeinrichtung, die einen an dem oberen Ende der Säule 7 angeordneten Elektromotor 14 aufweist, und eine Exzenterbetätigungseinrichtung vorgesehen.

Die Exzenterbetätigungseinrichtung umfaßt einen Exzenter 16 auf der Welle 15 des Elektromotors 14 und ein Rad 18 auf einer Achse 17, die mit dem stabförmigen Körper des Niederhalters 4a (Fig. 1) verbunden ist. Wenn der Elektromotor 10 einen geeigneten Niederhalter, beispielsweise den Niederhalter 4a, in die Betriebsposition in der Prüfeinrichtung gebracht hat, befindet sich das Rad 18 dieses Niederhalters 4a in einer Position, daß es durch den Exzenter 16 betätigbar ist. Das Rad 18 wird gegen den Exzenter 16 durch eine Rückholfeder 20a gedrückt, die zwischen einer Schulter 19a, die zwischen einem Abschnitt mit größerem Durchmesser und einem Abschnitt mit kleinerem Durchmesser des stabförmigen Körpers des Niederhalters 1a gebildet wird, und einem topfförmigen Gehäuse 20a sitzt, das den einen kleineren Durchmesser aufweisenden Abschnitt des stabförmigen Körpers umgibt und an der Unterseite der Halterung 8 befestigt ist. Die Rückholfeder 20a drückt daher den Niederhalter nach oben und hält den Niederhalter in einer Ruhestellung. Der stabförmige Körper des Niederhalters 4a wird bei seiner vertikalen Bewegung durch das Gehäuse 20b und ein Führungsrohr 19 geführt, welches auf der Oberseite der Halterung 8 angeordnet ist. Bei einer Drehung des Exzenters 16 durch den Elektromotor 14 wird daher der Niederhalter 4a gegen die Kraft der Rückholfeder 20a nach unten bewegt, bis der Niederhalter an dem Prüfkörper angreift. Wenn der Exzenter 16 weiter gedreht wird, wird der Niederhalter 4a durch die Rückholfeder 20a wieder in seine Ruhestellung zurück angehoben. Zur Aufnahme der Achse 17 hat das Führungsrohr 19 einen Schlitz 21, dessen Länge der Strecke der senkrechten Bewegung des Niederhalters 4a entspricht.

Die Haltevorrichtung ist so ausgelegt, daß sie von einer Steuereinheit, beispielsweise einem Computer, betrieben werden kann. Um alle Funktionen der Haltevorrichtung automatisch durch eine Steuereinheit steuern zu können, ist eine Justiereinrichtung zum Einstellen der Nullstellung der Halterung 8 vorgesehen. Zu diesem Zweck ist ein Stopmagnet 22 auf dem Ständer 5 angeordnet, wobei der Anker des Stopmagnets in eine Ausnehmung 23 der Halterung 8 eingreifen kann. Die Nullstellung ist dann verwirklicht, wenn der Anker des Stopmagnets in die Ausnehmung 23 eingreift. Wenn die Nullstellung verifiziert ist, wird der Anker des Stopmagnets 22 wieder aus der Ausnehmung 23 abgezogen. Ebenfalls im Zusammenhang mit der automatischen Steuerung der Haltevorrichtung ist vorzugsweise eine Abtasteinrichtung vorgesehen, um die Positionierung der Niederhalter in der Betriebsstellung genau festzustellen und die Niederhalter dort zu positionieren. Der Positionsgeber weist einen Kontaktstift 24 an der Halterung 8 und einen zugeordneten Kontaktschalter 26 auf, der an dem Ständer 5 gegenüber und nahe bei dem Kontaktstift 24 angeordnet ist. Der Kontaktstift 24 betätigt den Kontaktschalter 26, beispielsweise einen Zungenschalter, wenn die Halterung 8 sich in ihrer Nullstellung befindet. Die Positioniereinrichtung weist ferner eine Vielzahl von Kontaktstiften 25 auf der Halterung 8 auf, wobei sich die Kontaktstifte 25 unter gleichem Abstand von der Säule 7 befinden. Es sind genau so viele Kontaktstifte 25 wie Niederhalter vorgesehen. Die Kontaktstifte 25 sind mit zugeordneten Kontaktschaltern 27, beispielsweise Zungenschaltern, zusammen, die auf dem Ständer 5 gegenüber und nahe bei den Kontaktstiften 25 angeordnet sind. Durch die Betätigung des Kontaktschalters 27 durch einen Kontaktstift 25 wird die Positionierung eines Niederhalters in der Arbeits- und Betriebsstellung verifiziert.

Fig. 2 zeigt eine Draufsicht auf die Halterung 8 ohne ihre Abdeckung 8a. In Fig. 2 befindet sich der Niederhalter 4a in der Arbeitsstellung, in der er von dem Exzenter 16 betätigbar ist. Da ein Teil des Motors 14 weggebrochen dargestellt ist, zeigt Fig. 2 auch den Elektromotor 10, der über die Kegelzahnräder 12 und 13 mit der Säule 7 in Antriebsverbindung steht. Jeder der Niederhalter 4a, 4b, 4c und 4d hat ein Rad 18, obwohl solch ein Rad in Fig. 2 nur im Zusammenhang mit den beiden Niederhaltern 4a und 4b gezeigt ist. Bei der tatsächlichen Ausführung der Haltevorrichtung sind sechs Niederhalter vorgesehen, von denen in Fig. 2 vier Niederhalter gezeigt sind.

Fig. 3 zeigt eine Seitenansicht von einem Typ eines Niederhalters 4a, der an seinem unteren Ende Greifer 28 aufweist, die aus zwei winkelförmigen Backen 30a, 30b bestehen, die an ihren oberen Schenkeln um eine Drehachse 29 schwenkbar gelagert sind. Dadurch können sich die Backen 30a, 30b in Richtung der Pfeile B und C nach außen bewegen, wenn die Backen in Kontakt mit einem Prüfkörper 1a kommen. Da die Backen 30a, 30b eine gewisse Ausdehnung in Richtung senkrecht zu der Fig. 3 haben, wie aus Fig. 1 zu ersehen ist, umfassen die Backen den Prüfkörper 1a beim Absenken teilweise, richten ihn aus und halten ihn fest, wenn sie aufgrund der Schwerkraft durch den Prüfkörper 1a auseinander bewegt werden. Die Backen 30a, 30b halten somit aufgrund der Schwerkraft den Prüfkörper in der richtigen Position zwischen dem Anschlag 2 und dem Stempel 3. Es ist auch möglich, zusätzlich eine Feder (nicht gezeigt) zwischen den Backen 30a, 30b anzuordnen, so daß der Prüfkörper 1a fester erfaßt wird. Die Breite der Backen in Richtung senkrecht zu Fig. 3 sowie die gegebenenfalls vorgesehene Feder werden je nach dem Typ des zu haltenden Prüfkörpers ausgewählt. Bei dem bevorzugten Ausführungsbeispiel der Haltevorrichtung, das in den Zeichnungen dargestellt ist, sind sechs verschiedene Arten von Niederhaltern vorgesehen, wobei fünf Niederhalter mit fünf verschiedenen Greifereinrichtungen ausgestattet sind, die auf fünf verschiedene Typen von Prüfkörpern abgestimmt sind. Der letzte Niederhalter, der als Niederhalter 4b in Fig. 4 gezeigt ist, hat dann an seinem unteren Ende ein Kissen 31 aus elastischem Material um linsenförmige, kugelförmige oder flache, zylindrische Prüfkörper zu halten.

**Patentansprüche**

1. Haltevorrichtung für Prüfkörper, insbesondere Tabletten und dergleichen, in einer Prüfeinrichtung, in der die Bruchfestigkeit der Prüfkörper gemessen wird, enthaltend:

eine Trageeinrichtung (57), die eine bewegbare Halterung (8) trägt, auf der eine Vielzahl von Niederhaltern (4a, 4b . . . ) angeordnet sind, die jeweils Prüfkörpern (1a, 1b . . . ) mit verschiedenen Formen zugeordnet sind und diese auf einer Grundplatte (6) festhalten und/oder ausrichten,

eine Antriebseinrichtung (10 - 13), die die Halterung derart bewegt, daß sie einen für den jeweiligen Prüfkörper (1a, 1b . . . ) geeigneten Niederhalter (4a, 4b . . . ) in eine Arbeitsposition bringt, und eine Absenkeinrichtung (14 - 18), die jeweils den in der Arbeitsposition befindlichen Niederhalter (4a, 4b . . . ) auf die Grundplatte (6) absenkt, um den Prüfkörper (1a, 1b . . . ) festzuhalten und/oder auszurichten.

2. Haltevorrichtung nach Anspruch 1, wobei die Halterung (8) auf der Trageeinrichtung (5, 7) drehbar angeordnet ist, und die Niederhalter (4a, 4b . . . . ) kreisförmig an der Halterung (8) angeordnet sind.

3. Haltevorrichtung nach Anspruch 1 oder 2, wobei die Antriebseinrichtung (10 - 13) einen an der Halterung 8 angeordneten Elektromotor (10) enthält, der über ein Getriebe (12, 13) mit der Trageeinrichtung (5, 7) verbunden ist.

4. Haltevorrichtung nach Anspruch 3, wobei das Getriebe (12, 13) als Kegelradgetriebe ausgebildet ist, das ein an der Welle (11) des Elektromotors (10) angeordnetes Kegelrad (12) und ein an einer Säule (7) der Trageeinrichtung (5, 7) angeordnetes Kegelrad (13) enthält.

5. Haltevorrichtung nach einem der Ansprüche 1 bis 4, wobei die Absenkeinrichtung (14 - 18) einen an der Trageeinrichtung (5, 7) angeordneten Elektromotor (14) und einen an dessen Welle (15) angeordneten Exzenter (16) und ein an diesem anliegendes, am Niederhalter (4a, 4b . . . . ) angeordnetes Rad (18) enthält.

6. Haltevorrichtung nach einem der Ansprüche 1 bis 5, wobei jeder Niederhalter (4a, 4b . . . . ) eine Rückstellfeder (20a) enthält, die ihn in eine Ruhestellung anhebt und hält.

7. Haltevorrichtung nach einem der Ansprüche 1 bis 6, wobei mindestens einer der Niederhalter (4a, 4b . . . ) an seinem unteren Ende eine Greifeinrichtung (28) aufweist, die zwei winkelförmige Backen (30a, 30b) aufweist, die an ihrem oberen Schenkel um eine Drehachse (29) schwenkbar gelagert sind.

8. Haltevorrichtung nach einem der Ansprüche 1 bis 7, wobei mindestens einer der Niederhalter (4a, 4b . . . ) an seinem unteren Ende ein Kissen (31) aufweist, das den Prüfkörper gegen die Grundplatte (6) drückt.

9. Haltevorrichtung nach einem der Ansprüche 1 bis 8, wobei an der Halterung (8) und an der Trageeinrichtung (5, 7) Positionsgeber (24 - 27) angeordnet sind.

10. Haltevorrichtung nach einem der Ansprüche 1 bis 9, wobei an der Trageeinrichtung (5, 7) ein Stopmagnet (22) vorgesehen ist, dessen Anker in eine Ausnehmung (23) der Halterung (8) eingreift.

**Revendications**

1. Dispositif de retenue d'éprouvettes, en particulier des comprimés et objets similaires, dans un dispositif de contrôle dans lequel la résistance des éprouvettes à la rupture est mesurée, comprenant:

un dispositif de support (5, 7) portant un élément mobile de retenue (8), sur lequel est installé un grand nombre de serre flans (4a, 4b . . . ) qui sont respectivement associés à des éprouvettes (1a, 1b . . . ) de formes différentes, et assujettissent et/ou orientent ces dernières sur une plaque de base (6),

un dispositif d'entraînement (10-13) qui imprime, à l'élément de retenue, des mouvements tels qu'il amène à une position de travail un serre-flans (4a, 4b . . . ) approprié pour l'éprouvette considérée (1a, 1b . . . ), et

un dispositif d'abaissement (14-18) qui abaisse, sur la plaque de base (6), le serre-flans (4a, 4b . . . ) occupant respectivement la position de travail, afin d'assujettir et/ou d'orienter l'éprouvette (1a, 1b . . . ).

2. Dispositif de retenue selon la revendication 1, dans lequel l'élément de retenue (8) est installé à rotation sur le dispositif de support (5, 7), et les serre-flans (4a, 4b . . . ) sont agencés circulairement sur l'élément de retenue (8).

3. Dispositif de retenue selon la revendication 1 ou 2, dans lequel le dispositif d'entraînement (10-13) renferme un moteur électrique (10) installé sur l'élément de retenue (8), et relié au dispositif de support (5, 7) par l'intermédiaire d'une transmission (12, 13).

4. Dispositif de retenue selon la revendication 3, dans lequel la transmission (12, 13) est réalisée sous la forme d'une transmission par pignons coniques comprenant un pignon conique (12) disposé sur l'arbre (11) du moteur électrique (10), ainsi qu'un pignon conique (13) disposé sur une colonne (7) du dispositif de support (5, 7).

5. Dispositif de retenue selon l'une des revendications 1 à 4, dans lequel le dispositif d'abaissement (14-18) renferme un moteur électrique (14) installé sur le dispositif de support (5, 7), un excentrique (16) disposé sur l'arbre (15) dudit moteur, ainsi qu'un pignon (18) qui est appliqué contre ledit excentrique et est disposé sur le serre-flans (4a, 4b . . . ).

6. Dispositif de retenue selon l'une des revendications 1 à 5, dans lequel chaque serre-flans (4a, 4b . . . ) renferme un ressort de rappel (20a), qui le soulève à une position de repos et l'y maintient.

7. Dispositif de retenue selon l'une des revendications 1 à 6, dans lequel au moins l'un des serre-flans (4a, 4b . . . ) comporte, à son extrémité inférieure, un dispositif de préhension (28) comprenant deux mâchoires coudées (30a, 30b) qui sont montées pivotantes, par leur branche supérieure, autour d'un axe de rotation (29).

8. Dispositif de retenue selon l'une des revendications 1 à 7, dans lequel au moins l'un des serre-flans (4a, 4b . . . ) présente, à son extrémité inférieure, un coussin (31) qui presse l'éprouvette contre la plaque de base (6).

9. Dispositif de retenue selon l'une des revendications 1 à 8, dans lequel des indicateurs de positions (24-27) sont implantés sur l'élément de retenue (8) et sur le dispositif de support (5, 7).

10. Dispositif de retenue selon l'une des revendications 1 à 9, dans lequel un aimant d'arrêt (22) est prévu sur le dispositif de support (5, 7), aimant dont l'induit pénètre dans un évidement (23) de l'élément de retenue (8).

**Claims**

1. A holding device for test samples, in particular tablets and the like, in a testing station in which the breaking strength of the test samples is measured, comprising:

a supporting structure (5, 7) carrying a movable mounting support (8) on which a plurality of press pads (4a, 4b...) are arranged, which press pads are adapted to the different shapes of the respective test samples (1a, 1b . . . ) and hold and/or align said test samples on a base plate (6),

a drive means (10-13) for moving said mounting support in such manner that the press pads (4a, 4b . . . ) suitable for the respective test samples (1a, 1b . . . ) are brought into an operating position, and lowering means (14-18) for lowering the respective press pad (4a, 4b . . . ) just being in the operating position down to the base plate (6) in order to hold and/or align said test sample (1a, 1b . . . ).

2. A holding device according to claim 1, wherein the mounting support (8) is rotatably supported on the supporting structure (5, 7) and the press pads (4a, 4b . . . ) are circularly arranged on the mounting support (8).

3. A holding device according to claim 1 or 2, wherein the drive means (10-13) comprises an electric motor (10) mounted on the mounting support (8) and connected to the supporting structure (5, 7) by means of a gearing (12, 13).

4. A holding device according to claim 3, wherein the gearing (12, 13) is a bevel gearing containing a bevel gear (12) arranged at the shaft (11) of the electric motor (10) and a bevel gear (13) arranged on a column (7) of the supporting structure (5, 7).

5. A holding device according to any of the claims 1 to 4, wherein the lowering means (14-18) comprises an electric motor (14) arranged on the supporting structure (5, 7), an eccentric (16) arranged on the shaft (15) of said motor, and a wheel (18) adjacent to said eccentric and arranged on the press pad (4a, 4b . . . ).

6. A holding device according to any of the claims 1 to 5, wherein each press pad (4a, 4b . . . ) contains a return spring (20a) for lifting and holding said press pad in an idle position.

7. A holding device according to any of the claims 1 to 6, wherein at least one of the press pads (4a, 4b . . . ) has, at its lower end, a gripper means (28) comprising two angular jaws (30a, 30b) which are at their upper legs swingably attached to a rotation axis (29).

8. A holding device according to any of the claims 1 to 7, wherein at least one of the press pads (4a, 4b . . . ) has, at its lower end, a cushion (31) pressing the test sample against the base plate (6).

9. A holding device according to any of the claims 1 to 8, wherein positioning devices (24-27) are disposed at the mounting support (8) and at the supporting structure (5, 7).

10. A holding device according to any of the claims 1 to 9, wherein the supporting structure (5, 7) comprises a stop magnet (22), the anchor of which engages a recess (23) in the mounting support (8).

**FIG. 1**

# FIG. 2

# FIG. 3

# FIG. 4

2